# EUROPÄISCHE PATENTSCHRIFT

(11) **EP 2 604 179 B1**
(45) Veröffentlichungstag und Bekanntmachung des Hinweises auf die Patenterteilung: **24.09.2014**
(21) Anmeldenummer: 11401667.8
(22) Anmeldetag: 15.12.2011
(51) Int. Cl.: A61B 1/12, B08B 9/032

(54) **Verfahren zur Reinigung von englumigen Gerätschaften**
Method for cleaning narrow-necked equipment
Procédé de nettoyage d'outils à lumière

(43) Veröffentlichungstag der Anmeldung: 19.06.2013
(73) Patentinhaber: Miele & Cie. KG, 33332 Gütersloh (DE)
(72) Erfinder: Mracek, Maik, 33334 Gütersloh (DE); Penner, Markus, 32791 Lage (DE)

(56) Entgegenhaltungen:
- DE-A1- 3 308 729
- DE-A1- 19 545 528
- JP-A- 1 067 289
- JP-A- 2003 024 892
- JP-A- 2004 275 561

## Beschreibung

Die Erfindung betrifft ein Verfahren zur Reinigung von englumigen Gerätschaften, insbesondere von Endoskopen, MIC-Instrumenten und/oder dergleichen medizinischen Gerätschaften, bei dem eine Reinigungsflüssigkeit in einen Kanal der zu reinigenden Gerätschaft eingeleitet und durch diesen hindurchgeführt wird.

In der Medizintechnik kommen verstärkt mikroinvasive Operationstechniken zum Einsatz. Hierbei finden entsprechende medizinische Gerätschaften Verwendung, wie zum Beispiel Endoskope oder Gerätschaften der sogenannten minimalinvasiven Chirurgie, kurz MIC.

Bei derlei Gerätschaften handelt es sich um englumige Gerätschaften, das heißt um Gerätschaften mit Schläuchen, Kanälen und/oder dergleichen, die über geringe Innendurchmesser verfügen. Dabei sind unter "geringe Innendurchmesser" im Sinne der Erfindung Durchmesser in einer Größenordnung von ca. 0,1 mm bis 4 mm zu verstehen.

Im Zuge einer bestimmungsgemäßen Verwendung englumiger Gerätschaften der vorgenannten Art kommt es zu einer Verschmutzung derselben, beispielsweise durch Blutanlagerung. Vor einem erneuten Einsatz sind die Gerätschaften deshalb zu reinigen und zu desinfizieren.

Aus dem Stand der Technik sind zweistufige Verfahren zur Reinigung von englumigen Gerätschaften bekannt. Dabei erfolgt gemäß einer ersten Reinigungsstufe eine Vorreinigung mittels einer Bürste, die manuell, das heißt von Hand durch die zu reinigenden Lumen hindurchzuführen ist. In einer zweiten Reinigungsstufe erfolgt sodann eine Spülung mit einer Reinigungsflüssigkeit, beispielsweise Wasser. Dabei wird die Reinigungsflüssigkeit mit erhöhtem Druck von zum Beispiel bis zu 3 bar und mehr durch die querschnittsengen Kanäle, das heißt die Lumen hindurchgeführt. In der ersten Reinigungsstufe durch mechanische Einwirkung aufgebrochene und abgelöste Verschmutzungen können so ausgetrieben werden.

Obgleich sich das vorbeschriebene Verfahren zur Reinigung von englumigen Gerätschaften im alltäglichen Praxiseinsatz bewährt hat, besteht Verbesserungsbedarf. So wird es insbesondere als nachteilig empfunden, dass das Reinigungsergebnis von der manuell durchzuführenden Vorreinigung abhängig ist und somit reproduzierbare Reinigungsergebnisse nicht erzielt werden können. Darüber hinaus ist die Reinigung aufgrund der manuellen Vorbehandlung zeit- und damit kostenintensiv. Zudem kann es infolge der Bürstenvorreinigung durch mechanischen Abtrag zu Riefenbildungen an der Innenoberfläche der Volumen kommen, so dass sich nicht nur ein erhöhter Verschleiß einstellt, sondern sich auch eine vergrößerte Wirkoberfläche ergibt, an der Verschmutzungen, Keime, Bakterien und/oder dergleichen anhaften können.

Die JP2004275561A offenbart ein Verfahren zur Reinigung von Endoskopen, bei dem eine Reinigungsflüssigkeit und ein Luftstrom in einen Kanal des zu reinigenden Endoskops eingeleitet wird, wobei die Einleitung der Reinigungsflüssigkeit pulsierend erfolgt derart, dass sich insgesamt eine wellige Strömung im Endoskop einstellt.

Es ist ausgehend vom Vorbeschriebenen die Aufgabe der Erfindung, ein Verfahren zur Reinigung von englumigen Gerätschaften bereitzustellen, das bei gleichzeitig verbessertem Reinigungsergebnis einfacher durchzuführen ist.

Zur Lösung dieser Aufgabe wird mit der Erfindung ein Verfahren mit den Merkmalen von Anspruch 1 vorgeschlagen.

Im Unterschied zum Stand der Technik wird mit der Erfindung ein Verfahren vorgeschlagen, demgemäß nicht nur eine Reinigungsflüssigkeit in Form von z. B. Wasser durch die englumigen Kanäle der zu reinigenden Gerätschaft hindurchgeführt wird. Es sind vielmehr Reinigungsflüssigkeitsschübe einerseits und Luftschübe andererseits vorgesehen, die abwechselnd aufeinander nachfolgend, das heißt alternierend durch den englumigen Kanal hindurchgeführt werden. Zu diesem Zweck ist erfindungsgemäß vorgesehen, dass die Zuführung mit Reinigungsflüssigkeit mehrfach, vorzugsweise in regelmäßigen Abständen unterbrochen wird. Sobald die Zufuhr mit Reinigungsflüssigkeit unterbrochen ist, wird anstelle der Reinigungsflüssigkeit Luft in den englumigen Kanal eingeführt. Nach Abschluss der Luftzuführung erfolgt wieder eine Zufuhr von Reinigungsflüssigkeit. Diese abwechselnde Zufuhr von Luft und Reinigungsflüssigkeit wird wiederholt durchgeführt, so dass in der Konsequenz Reinigungsflüssigkeit und Luft alternierend durch den zu reinigenden Kanal hindurchgeführt werden.

Die erfindungsgemäße Verfahrensdurchführung erweist sich in vielerlei Hinsicht als vorteilhaft. Durch die abwechselnde Zuführung des zu reinigenden Kanals mit Reinigungsflüssigkeit einerseits und Luft andererseits wird ein gegenüber dem Stand der Technik derart verbessertes Reinigungsergebnis erreicht, dass auf eine manuelle Vorreinigung mittels Bürsten vollends verzichtet werden kann. Das erfindungsgemäße Verfahren erweist sich damit als weniger zeitaufwendig in der Durchführung und damit als kostengünstiger. Darüber hinaus kann ein reproduzierbares Verfahrensergebnis erreicht werden, da es auf eine individuelle Geschicklichkeit bei der manuellen Vorreinigung nicht weiter ankommt.

Das aufgrund der erfindungsgemäßen Verfahrensdurchführung verbesserte Verfahrensergebnis lässt sich im Wesentlichen auf zwei Gründe zurückführen. Aufgrund der alternierenden Zuführung von Reinigungsflüssigkeit einerseits und Luft andererseits entstehen Phasengrenzen zwischen den einzelnen Flüssigkeits- und Luft-"Paketen". Diese Phasengrenzen bewirken eine mechanische Beanspruchung der Innenoberfläche der zu reinigenden Lumen, infolge dessen es zu einem Schmutzabtrag kommt. Ein zweiter Grund sind die sich im Reinigungsmedium insgesamt ergebenden Druckschwankungen. Gemäß der Erfindung setzt sich das Reinigungsmedium aus der Reinigungsflüssigkeit einerseits und der Luft andererseits zusammen. Dabei stellt der Luftanteil einen insgesamt kompressiblen Anteil dar, wohingegen die Reinigungsflüssigkeit annähernd inkompressibel ist. Infolge dessen kommt es bei einer Einführung des Reinigungsmediums in die englumigen Kanäle zu Relativbewegungen zwischen den Reinigungsflüssigkeits-"Paketen" einerseits und den Luft-"Paketen" andererseits. Diese Dynamik trägt ebenfalls zu einem verbesserten Reinigungsergebnis bei.

Gegenüber der nach dem Stand der Technik notwendigen Vorbehandlung mittels Bürsten stellt sich gemäß dem erfindungsgemäßen Verfahren ferner der Vorteil ein, dass Rückstände vollends herausgespült werden, anders als beim Bürsten, demgemäß aufgrund der Bürstenbewegung nur ein Hin- und Herbewegen abgelöster Rückstände erfolgt. Darüber hinaus wird ein Verschleiß der Innenoberfläche der englumigen Kanäle durch eine Bürstenbeaufschlagung vollends vermieden.

Darüber hinaus ist von Vorteil, dass das erfindungsgemäße Verfahren aufgrund des zumindest teilweisen Ersatzes von Reinigungsflüssigkeit durch Luft weniger Reinigungsflüssigkeit als aus dem Stand der Technik bekannte Verfahren benötigt.

Ein weiterer Vorteil des erfindungsgemäßen Verfahrens ist die optimierte Einstellung der Verfahrensparameter auf die zu reinigenden Gerätschaften, insbesondere auf die Innendurchmessergrößen der englumigen Kanäle. In diesem Zusammenhang ist ferner von Vorteil, dass das erfindungsgemäße Verfahren auch dazu geeignet ist, englumige Kanäle zu reinigen, die über einen Ringspalt verfügen. In derlei Ringspalte können keine Bürsten eingeführt werden, so dass aus dem Stand der Technik vorbekannte Verfahren dem Grunde nach nicht dazu geeignet sind, derlei ausgebildete Kanäle reinigen zu können. Das erfindungsgemäße Verfahren schafft hier Abhilfe.

Gemäß einem weiterem Merkmal der Erfindung ist vorgesehen, Verfahrensparameter zu Beginn der Verfahrensdurchführung und/oder während der Verfahrensdurchführung einzustellen. Als Verfahrensparameter kommen dabei insbesondere das Luft-Reinigungsflüssigkeit-Verhältnis, der Luftdruck und/oder der Reinigungsflüssigkeitsdruck sowie die Frequenz der Lufteinleitung in Betracht. Dabei können die vorgenannten Verfahrensparameter einzeln oder in Kombination eingestellt werden.

Typischerweise erfolgt während der Verfahrensdurchführung eine Beschickung des zu reinigenden Kanals mit Reinigungsflüssigkeit und anschließend eine Beschickung mit Luft, wobei sich diese Beschickung in vorbeschriebener Weise abwechselt. Dabei wird die Beschickungsdauer, das heißt die Zeitdauer der Beschickung durch die Öffnungszeit der entsprechenden Ventile bestimmt. Je länger die Öffnungszeit ist, desto größer der zugeführte Lumenstrom. Bevorzugt ist dabei grundsätzlich, dass im Verhältnis mehr Luft als Reinigungsflüssigkeit zugeführt wird. Dabei erfolgt die diesbezüglich Abstimmung hinsichtlich des Luft-Reinigungsflüssigkeit-Verhältnisses bevorzugterweise in Abhängigkeit des Durchmessers des englumigen Kanals. Gewählt wird erfindungsgemäß ein Zeitdauerverhältnis zur Einleitung der Reinigungsflüssigkeit einerseits und der Luft andererseits von 0,01s bis 0,5s (Reinigungsflüssigkeit) zu 0,1s bis 1,5s (Luft). Besonders vorgezogenerweise steht die Zeitdauer der Beschickung mit Reinigungsflüssigkeit zur Zeitdauer der Beschickung mit Luft in einem relativen Verhältnis im Bereich von 1 zu 1 bis 1 zu 100, vorzugsweise in einem relativen Verhältnis im Bereich von 1 zu 10, demgemäß wird vorzugsweise im Verhältnis wesentlich mehr Luft als Reinigungsflüssigkeit zugeführt. Die Frequenz der Lufteinleitung liegt vorzugsweise im Bereich zwischen 0,6 bis 10 Hz.

Bevorzugterweise wird die alternierende Beschickung des zu reinigenden Kanals, d.h. die Hindurchführung der Reinigungsflüssigkeit einerseits und der Luft anderseits insgesamt, d.h. über die mehrfachen Beschickungen mit Reinigungsflüssigkeit und Luft summiert, mindestens über einen Zeitraum von 30 Sekunden aufrechterhalten, vorzugsweise hat der Zeitraum eine Länge von 30 Sekunden bis 10 Minuten, besonders bevorzugt ist eine Länge zwischen 3 und 7 Minuten. Somit erfolgt insgesamt in jedem Fall vielfach eine Einleitung von Luft in den Kanal, d.h. ein Luftschub, vorzugsweise mindestens 100 einzelne Luftschübe.

Mit der Erfindung wird desweiteren eine Vorrichtung vorgeschlagen, und zwar eine Vorrichtung zur Reinigung von englumigen Gerätschaften, insbesondere von Endoskopen, MIC-Instrumenten und/oder dergleichen medizinische Gerätschaften, mit einem Anschluss für einen Kanal der zu reinigenden Gerätschaft, wobei der Anschluss an eine Zuführungsleitung für Reinigungsflüssigkeit einerseits und an eine Zuführungsleitung für Luft andererseits angeschlossen ist, wobei die beiden Zuführungsleitungen jeweils ein Ventil aufweisen, welche beiden Ventile alternierend betätigbar sind.

Gemäß der erfindungsgemäßen Vorrichtung ist der zu reinigende englumige Kanal an einen Anschluss der Vorrichtung anzuschließen. Sobald dies erfolgt ist, kann eine Beschickung des Kanals zum Zwecke der Reinigung mit Reinigungsmedium erfolgen, wobei das Reinigungsmedium Reinigungsflüssigkeit einerseits sowie Luft andererseits umfasst, wobei die Reinigungsflüssigkeit und die Luft alternierend, das heißt in Paketen oder Schüben in den Kanal eingeleitet werden. Zu diesem Zweck ist vorrichtungsseitig vorgesehen, dass der Anschluss einerseits an eine Zuführungsleitung für die Reinigungsflüssigkeit und anderseits an eine Zuführungsleitung für Luft angeschlossen ist. Dabei verfügen die beiden Zuführungsleitungen jeweils über ein Ventil, mittels welchem die zugehörige Zuführungsleitung verschließbar ist. Im bestimmungsgemäßen Anwendungsfall werden die beiden Ventile abwechselnd, das heißt alternierend betätigt, so dass in den Kanal in der schon vorbeschriebenen Weise abwechselnd Reinigungsflüssigkeitsschübe und Luftschübe einströmen. Die mit einer solchen Vorrichtung erzielten Vorteile entsprechen den schon zum erfindungsgemäßen Verfahren vorerläuterten Vorteilen.

Gemäß einer besonderen Ausführungsform ist das Ventil der Zuführungsleitung für die Reinigungsflüssigkeit ein Rückschlagventil. Diese Art der Ausgestaltung erweist sich als insbesondere kostengünstig.

Die Vorrichtung nach der Erfindung kann gemäß einem weiteren Merkmal der Erfindung über eine Mehrzahl von Anschlüssen verfügen. Dabei dient ein jeder Anschluss der Verbindung zu einem zu reinigenden Kanal. Aus dem Stand der Technik bekannte Endoskope können über bis zum 8 Kanäle aufweisen. Bevorzugterweise verfügt die erfindungsgemäße Vorrichtung deshalb über mindestens 8 Anschlüsse. Dabei ist ein jeder der mindestens 8 Anschlüsse an eine Wasserzuführung und an eine Luftzuführung angeschlossen.

Gemäß einem weiteren Merkmal der Erfindung ist vorgesehen, dass die Anschlüsse hinsichtlich der Reinigungsflüssigkeit ausgangsseitig an eine gemeinsame Verteileinrichtung angeschlossen sind. Gemäß dieser Ausführungsform strömt über eine gemeinsame Leitung Reinigungsflüssigkeit in die Verteileinrichtung. Von hier aus findet dann eine Verteilung auf die einzelnen Anschlüsse der Vorrichtung statt.

Die Verteileinrichtung verfügt gemäß einem weiteren Merkmal der Erfindung über einen Filter. Die vorzugsweise im Umwälzbetrieb verwendete Reinigungsflüssigkeit passiert so vor einem Einströmen in die jeweiligen Anschlüsse den in der Verteileinrichtung vorhandenen Filter. Es findet folglich nicht eine Filterung je Anschluss sondern eine Filterung in der Verteileinrichtung statt, so dass gefilterte Reinigungsflüssigkeit auf die einzelnen Anschlüsse verteilt wird.

Gemäß einem weiteren Merkmal der Erfindung ist je Anschluss eine Drucksensoreinrichtung vorgesehen. Mittels dieser Drucksensoreinrichtung ist ein automatisierter Betrieb der erfindungsgemäßen Vorrichtung möglich. So wird es durch die Drucksensoreinrichtung gestattet, einerseits das Vorhandensein eines zu reinigenden Kanals zu detektieren und andererseits festzustellen, welchen Innendurchmesser dieser zu reinigende Kanal besitzt. Die für bestimmte Kanalinnendurchmesser vorgegebenen Parameter können dann automatisiert eingestellt und der Reinigungsvorgang begonnen werden.

Weitere Vorteile und Merkmale der Erfindung ergeben sich aus der nachfolgenden Beschreibung anhand der Figuren. Dabei zeigen
- Figur 1: in einer schematischen Darstellung anhand eines Blockdiagramms die erfindungsgemäße Funktionsweise und
- Figur 2: in einer schematischen Darstellung ein Ausführungsbeispiel nach der Erfindung sowie
- Figur 3: in einer schematischen Darstellung eine Prinzipskizze bezüglich der erfindungsgemäßen Verfahrensdurchführung.

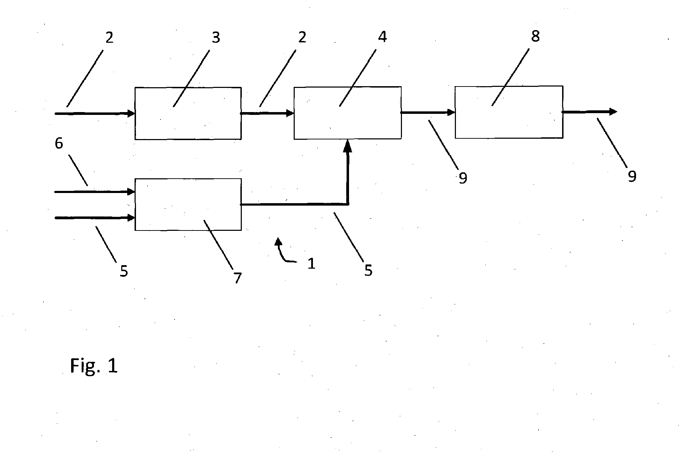

Figur 1 lässt die erfindungsgemäße Vorrichtung 1 sowie die Funktionsweise der erfindungsgemäßen Verfahrensdurchführung schematisch anhand eines Blockdiagramms erkennen.

Das Bezugszeichen 8 kennzeichnet einen zu reinigendes Lumen, das heißt einen zu reinigenden englumigen Kanal beispielsweise eines Endoskops. Zum Zwecke der Reinigung wird Reinigungsflüssigkeit in Form von zum Beispiel Wasser in Entsprechung des Pfeils 2 durch eine Pumpe 3 gefördert und einer Mischstelle 4 zugeführt. Der Mischstelle 4 wird desweiteren in Entsprechung des Pfeils 5 Luft über ein Ventil 7 zugeführt. Das Ventil 7 ist mittels elektrischer Signale 6 steuerbar.

Im bestimmungsgemäßen Verwendungsfall wird der Wasserstrom 2 mehrfach, etwa in regelmäßigen Abständen durch eingeblasene Druckluft unterbrochen. Der Druckbereich der Druckluft liegt bevorzugt zwischen 1 bar und 5 bar. Ausgangsseitig der Mischstelle 4 werden infolge dessen Wasser-Luft-"Pakete" 9, beziehungsweise -Schübe 9 zur Beschickung des englumigen Kanals 8 abgegeben.

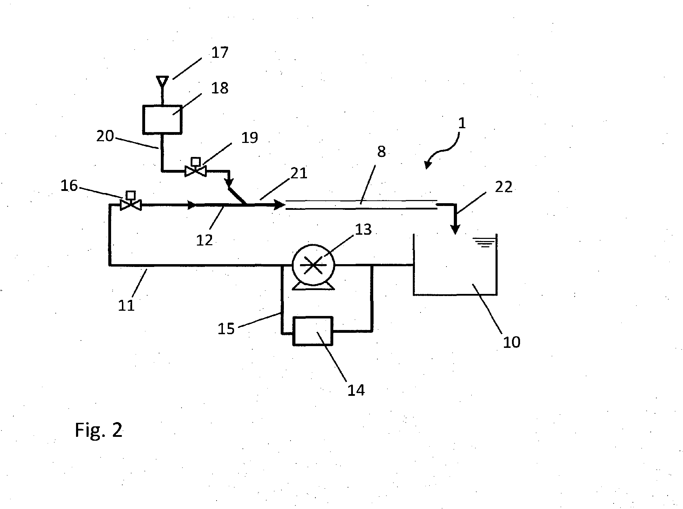

Figur 2 lässt anhand einer schematischen Darstellung eine bevorzugte Ausführungsform der Erfindung erkennen.

Die erfindungsgemäße Vorrichtung 1 verfügt über ein Wasserreservoir 10. An dieses Wasserreservoir 10 ist eine Wasser-Zuführungsleitung 11 angeschlossen. Über eine Umwälzpumpe 13 wird Wasser dem Wasserreservoir 10 entnommen und in die Zuführungsleitung 11 gefördert. Zum Zwecke der Aufrechterhaltung eines vorbestimmbaren Drucks ist ein Druckhalteregler 14 vorgesehen, der in eine zur Umwälzpumpe 13 ausgebildete Bypassleitung 15 integriert ist. Die Wasser-Zuführungsleitung 11 mündet unter Zwischenschaltung eines Wasserventils 16 in ein als Mischstelle 4 dienendes Y-Stück 12.

An das Y-Stück 12 ist eingangsseitig ferner eine Luft-Zuführungsleitung 20 angeschlossen. Diese verbindet das Y-Stück 12 unter Zwischenschaltung eines Druckluftreglers 18 und eines Luftventils 19 mit einer Druckluftquelle 17.

Der englumige Kanal 8 einer zu reinigenden Gerätschaft ist ausgangsseitig des Y-Stücks 12 angeschlossen. Ausgangsseitig steht der Lumen 8 mit dem Wasserreservoir 10 in strömungstechnischer Verbindung 22, wodurch der Kreislauf geschlossen ist.

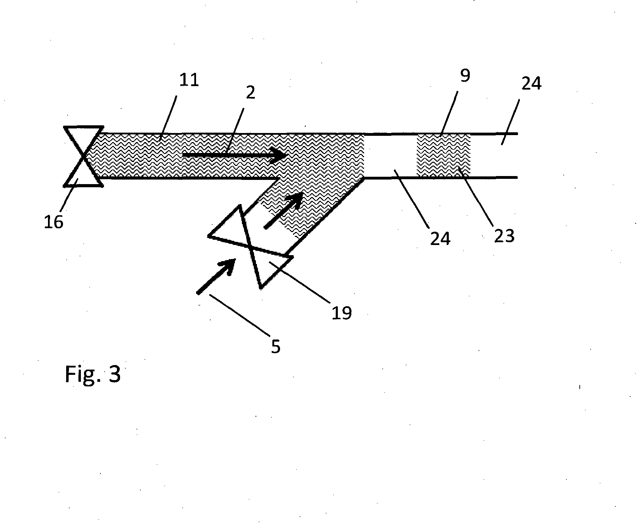

Im bestimmungsgemäßen Verwendungsfall wird über die Umwälzpumpe 13 Reinigungsflüssigkeit in Form von Wasser über die Wasser-Zuführungsleitung 11 zum Y-Stück 12 gefördert. Der Wasserstrom wird alternierend durch Einblasen von Luft unterbrochen, so dass einander abwechselnde Schübe 21 von Wasser 23 und Luft 24 zur Beschickung des Lumen 8 ausgebildet werden, wie dies Figur 3 erkennen lässt.

Bei der erfindungsgemäßen Verfahrensdurchführung sollten die angesetzten Strömungsgeschwindigkeiten vorzugsweise relativ hoch sein. Die Grenzschichten zwischen den einzelnen Wasser- beziehungsweise Luftschüben 23 und 24 sorgen dabei für Dynamik an den Kanalwandungen und führen so zu einem Mitreißen von Schmutzpartikeln. Zum Erreichen einer optimierten Reinigungsleistung können verschiedene Parameter des Verfahrens variiert werden. So kann einerseits das Luft-Wasser-Verhältnis verändert und andererseits die Druckverhältnisse angepasst werden. Als weitere Variation ist die Frequenz der Luftschübe variabel.

Zur weiteren Optimierung des Reinigungsergebnisses insbesondere von unterschiedlich großen Lumen 8 kann einen Messung der Lumeninnendurchmesser durchgeführt werden. Dies kann zum Beispiel mittels einem Spüldrucksensor erfolgen, der direkt an der Anschlussstelle für den Lumen 8 angebracht ist. Mit einem derartigen Messsystem kann bei Einleitung eines Mediums, das heißt von Luft oder Wasser aufgrund des anschließenden Druckabfalls an der angeschlossenen Gerätschaft, das heißt an dem angeschlossenen Kanal auf den Kanalinnendurchmesser zurückgeschlossen werden. Auf Basis dieses Messergebnisses kann dann automatisiert die optimierte Parametereinstellung vorgenommen werden.

### Bezugszeichenliste

- 1: Vorrichtung
- 2: Pfeil (Wasser)
- 3: Pumpe
- 4: Mischstelle
- 5: Pfeil (Luft)
- 6: elektrisches Signal
- 7: Ventil
- 8: Lumen
- 9: Pfeil (Wasser-Luft-Schübe)
- 10: Wasserreservoir
- 11: Zuführungsleitung (Wasser)
- 12: Y-Stück
- 13: Umwälzpumpe
- 14: Druckhalteregler
- 15: Bypassleitung
- 16: Wasserventil
- 17: Druckluftquelle
- 18: Druckluftregler
- 19: Luftventil
- 20: Zuführungsleitung (Luft)
- 21: Pfeil (Wasser-Luft-Schub)
- 22: Pfeil (Wasser)
- 23: Wasserschub
- 24: Luftschub

## Patentansprüche

1. Verfahren zur Reinigung von englumigen Gerätschaften, insbesondere von Endoskopen, MIC-Instrumenten und/oder dergleichen medizinischen Gerätschaften, bei dem eine Reinigungsflüssigkeit innerhalb eines geschlossenen Kreislaufs in einen Kanal der zu reinigenden Gerätschaft eingeleitet und durch diesen hindurchgeführt wird, wobei die Zuführung mit Reinigungsflüssigkeit mehrfach unterbrochen und, sobald die Zufuhr mit Reinigungsflüssigkeit unterbrochen ist, statt der Reinigungsflüssigkeit Luft in den Kanal eingeleitet wird, wobei nach beendeter Luftzuführung wieder eine Zufuhr von Reinigungsflüssigkeit erfolgt, so dass Reinigungsflüssigkeit und Luft alternierend in Form von Paketen oder Schüben durch den Kanal hindurchgeführt werden, wobei ein Zeitdauerverhältnis zur Einleitung der Reinigungsflüssigkeit einerseits und der Luft andererseits von 0,01s bis 0,5s zu 0,1 s bis 1,5s gewählt wird.

2. Verfahren nach Anspruch 1, **dadurch gekennzeichnet, dass** die Zuführung mit Reinigungsflüssigkeit in regelmäßigen Abständen unterbrochen wird.

3. Verfahren nach Anspruch 1 oder 2, **dadurch gekennzeichnet, dass** das Luft-Reinigungsflüssigkeit-Verhältnis eingestellt wird.

4. Verfahren nach Anspruch Anspruch 1, 2 oder 3, **dadurch gekennzeichnet, dass** der Luftdruck und/oder der Reinigungsflüssigkeitsdruck eingestellt werden/wird.

5. Verfahren nach einem der vorhergehenden Ansprüche, **dadurch gekennzeichnet, dass** die Frequenz der Lufteinleitung eingestellt wird.

## Claims

1. Method for cleaning narrow-necked equipment, in particular endoscopes, MIC instruments and/or similar medical equipment, in which a cleaning fluid in a closed circuit is introduced into a channel of the equipment to be cleaned and is guided therethrough, wherein the supply of cleaning fluid is interrupted several times and, as soon as the supply of cleaning fluid is interrupted, air is introduced into the channel instead of the cleaning fluid, wherein, when the supply of air has ended, cleaning fluid is again supplied so that cleaning fluid and air are alternately guided through the channel in the form of packets or thrusts, wherein a ratio of duration of, on the one hand, the introduction of the cleaning fluid and, on the other hand, of the air, of 0.01 s up to 0.5 s to 0.1 s up to 1.5 s is selected.

2. Method according to claim 1, **characterised in that** the supply of cleaning fluid is interrupted at regular intervals.

3. Method according to either claim 1 or claim 2, **characterised in that** the ratio of air to cleaning fluid is adjusted.

4. Method according to claim 1, 2 or 3, **characterised in that** the air pressure and/or the cleaning fluid pressure is adjusted.

5. Method according to any of the preceding claims, **characterised in that** the frequency of the introduction of air is adjusted.

## Revendications

1. Procédé de nettoyage d'équipements à goulot étroit, en particulier d'endoscopes, d'instruments MIC et d'équipements médicaux similaires, dans lequel un liquide de nettoyage à l'intérieur d'un circuit fermé est introduit dans un canal de l'équipement à nettoyer et conduit à travers ce canal, l'alimentation en liquide de nettoyage étant interrompue plusieurs fois et, dès que l'alimentation en liquide de nettoyage est interrompue, c'est de l'air qui est introduit dans le canal au lieu du liquide de nettoyage, une alimentation en liquide de nettoyage étant effectuée encore quand l'alimentation en air est terminée, de telle sorte que du liquide de nettoyage et de l'air sont conduits à travers le canal de façon alternée sous forme de paquets ou de charges, un rapport de durée pour l'introduction du liquide de nettoyage d'un côté et d'air d'un autre côté étant choisi comme étant de 0,01 s à 0,5 s / 0,1 s à 1,5 s.

2. Procédé selon la revendication 1, **caractérisé en ce que** l'alimentation en liquide de nettoyage est interrompue à intervalles réguliers.

3. Procédé selon la revendication 1 ou 2, **caractérisé en ce que** le rapport air-liquide de nettoyage est réglé.

4. Procédé selon la revendication 1, 2 ou 3, **caractérisé en ce que** la pression d'air et/ou la pression de liquide de nettoyage est/sont réglée(s).

5. Procédé selon une des revendications précédentes, **caractérisé en ce que** la fréquence de l'introduction d'air est réglée.
